(19)

Europäisches
Patentamt
European
Patent Office
Office européen
des brevets

(11) **EP 2 878 342 A1**

(12) **EUROPEAN PATENT APPLICATION**

(43) Date of publication:
**03.06.2015 Bulletin 2015/23**

(51) Int Cl.:
***A61Q 19/08*** (2006.01)   ***A61K 8/97*** (2006.01)
***A61K 35/10*** (2015.01)   ***A61K 8/96*** (2006.01)

(21) Application number: **13194683.2**

(22) Date of filing: **27.11.2013**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **Latvijas Universitate**
**1586 Riga (LV)**

(72) Inventors:
• **Silamikele, Baiba**
**LV-1030 Riga (LV)**

• **Ramata-Stunda, Anna**
**LV-1011 Riga (LV)**
• **Muiznieks, Indrikis**
**LV-1011 Riga (LV)**

(74) Representative: **Fortuna, Aleksandra**
**Foral Patent Law Offices**
**P.O.Box 98**
**Riga 1050 (LV)**

(54) **Method for extraction of peat active substances and use of their combination in skin regenerating cosmetic formulations**

(57)    The present invention relates to extraction of peat active substances and use of their combination as skin care active ingredients to target skin ageing. Object of invention is the combination of peat ethanol extract with humic acids isolated from peat as skin care active complex. The invented active complex is the combination of 0,5-1 volume % peat extract with 5-20 $\mu$g/ml humic acids. Antiradical activity is characteristic to peat extract isolated with the proposed method and extracts combination with humic acids have capability to reduce dermal fibroblast population doubling time in vitro by up to 50% during the first 24h.

EP 2 878 342 A1

**Description**

*Technical field*

This invention relates to cosmetic agents and compositions for the regeneration of mature skin and delay of skin ageing.

*Prior art*

Peat is an organic soil type formed as a result of incomplete decay and humification of remains of marsh plants under high humidity conditions. The organic matter of peat in 90% are humin, humic and fulvic acids (up to 40%), lignin, polysaccharides, lipids, pectines, hemicellulose and cellulose. Peat and its preparations have been long used in balneotherapy, traditional medicine and cosmetic procedures. There is a growing interest on research of humus matters all over the world and a body of evidence has collected to show broad potential of peat for development of pharmaceutical and cosmetic products. Peat-derived products are used in diverse cosmetic prescriptions. Different extracts from peat can be obtained using various extraction media and various extraction methods. Such extracts, containing bioactive peat-derived components, then can be used for cosmetic and pharmaceutical purposes. (Schepetkin et al., 2002, Beer et al., 2003, Mendez et al., 2004 Yamada et al., 2007).

One of the fractions that can also be extracted from peat is humic acids, which have attracted attention for their potential use in healthcare. Humic acids belong to a group of polymers with high-molecular-weight macromolecules. Chemical and spectroscopic analyses have revealed the presence of aromatic rings, phenolic hydroxyl, ketonyl, quinone carbonyl, carbocyl and alkoxyl groups in humic acids (Burges et al, 1964, Yang et al., 2004,). Although the exact composition of humic acids is unknown, it varies according to their extraction source (Orru et al., 2011). Chemical structure, composition, functional groups present in humic acids vary markedly depending on their origin and humification processes they have undergone. Studies on humic acid chemistry have revealed that aromatic rings, phenolic hydroxyl, ketone carbon, quinone carbonyl, carboxyl and alkoxyl groups are present in their molecules. The biological effects of humic acids differ due to variability in their chemical structure and physiochemical properties (Stepanova et al, 1996). HA have been used not only in cosmetics but also in healthcare as antimicrobial, immunomodulatory and antitumor agents as well as for treatment for skin bums. They have been reported to have also profibrinolytic and estrogenic activities (Mendez et al., 2004, Yamada et al., 2007). Humic axcids have been used externally for treatment of hematoma, phlebitis, osteoarthritis, osteochondrosis (Laub, 1999). In vitro and in vivo tests have shown that fulvic and humic acids increase number and activity of macrophages, neutrophils and killer T-cells (Riede et al., 1991, Laub, 1999,). A product for topical use claiming antiviral characteristics has been developed (Schiller et al., 1979).

An example of use of humic acids for therapeutic reasons is proposed in the EP 1115408 B1 - a product containing humic acids for treatment of bacterial and viral infections via stimulation of TH 1 and TH 2 lymphocytes is proposed.

A pharmaceutical formulation containing a peat-derived bioactive product, in the form of a gel, is prepared by combining a sterile alcoholic herb extract with sterile glycerol, a sterile aqueous solution of previously powdered peat-derived bioactive product and a sterile menthol solution is proposed in EP 0533865 B1.

Object of invention in EP 0281679 B1 is the method for preparation of alkali huminates from humic matter and use of them as remedy in wound healing and preparation of highly effective synthetic mud baths.

A peat extract invented produced by carbon dioxide extraction (WO 2004050208 A1) has been proposed for diverse cosmetic prescriptions to improve skin moisture, decreasing skin sensitivity and inflammation, slowing down skin aging and improving overall health condition of skin, specified peat extract can be used as single bioactive ingredient or in combination with other ingredients such as natural and/or synthetic antioxidants and vitamins.

A cosmetic composition for skin cleansers and creams has been proposed, that along other ingredients, contain water-extract of peat as a bioactive component and dry powdered peat (Patent application No. DE 10025622 A1).

*Disclosure of the invention*

The present invention relates primarily to novel peat-derived bioactive products and to cosmetic compositions containing active complexed of peat derived bioactive compounds. More specifically a combination of peat ethanol extract and peat humic acid preparation is proposed as an active anti-ageing cosmetic ingredient.

An anti-ageing cosmetic active ingredient is proposed, being a peat derived bioactive product, characterized in that it contains peat extract in combination with humic acids isolated from peat. The peat extract is obtained by extraction with 80% ethanol with peat to ethanol ratio 1:100. The humic acids is obtainable by reacting dry peat with 0.1 M HCl, followed by resuspension of precipitate in water and adjustment of pH to 7 with the addition of 1 M NaOH, after 24 h the alkaline slurry is filtered and the particle free filtrate is acidified to a pH 1, then sediment which contained humic acids is washed with distilled water and centrifuged discarding the supernatant and humic acid dispersion in distilled water is dialyzed against water, resulting humic acids is lyophilized. The proposed active ingredient is capable to promote proliferation of dermal fibroblasts by 35-50% during the first 24 h of incubation. The proposed active ingredient can be used in anti-ageing cosmetic formulations at concentrations 0,5-1 volume % of peat extract combined with 5-20 μg/ml humic acids.

*Brief description of drawings*

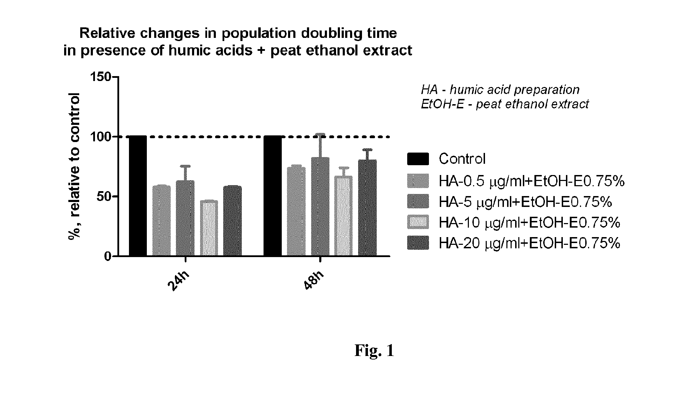

Fig. 1 is a graph showing an effect of combination of peat extract and humic acids on proliferation of dermal fibroblasts.

The ethanol extract was made adding 80% ethanol to dry peat. Peat to ethanol ratio was 1:100. Extraction was performed by shaking the suspension at speed 160 rpm for 24h at room temperature. Extraction was followed by filtration through filter paper to separate peat particles from the extract, afterwards peat extract was sterile filtered through 0.45 μm syringe filter.

For humic acid isolation 10.0 g of air dry peat was reacted with 200 ml of 0.1 M HCl for 1 h. The slurry was allowed to settle and the aqueous phase was decanted and discarded. Approximately 100 ml of water was added to the peat mass and the resulting slurry was allowed to incubate for 30 min, after which the pH of the slurry was adjusted to 7 with the addition of 1 M NaOH. This was followed by the addition of a sufficient quantity of 1 M NaOH, to bring the total volume of the solution phase to 2 liters. After 24 h the alkaline slurry was filtered through glasswool and the particle free filtrate was acidified to a pH 1, with the addition of 6 M HCl. This solution was centrifuged and the supernatant was discarded. The sediment (which contained humic acids) was washed with distilled water and repeatedly centrifuged discarding the supernatant. Afterwards humic acid dispersion in distilled water were dialyzed against water and resulting humic acids were lyophilized. Lyophilized humic acids were dissolved in 0.01M NaHCO$_3$.

Chemical and biological activities of peat extract, humic acid preparation and their combinations were tested:

Antiradical activity of peat extract

The method consisted of spectrophotometric measurement of the intensity of the color change in solution depending on the amount of 2,2-diphenyll-picrylhydrazyl (DPPH). The reaction was initiated by mixing 1 mL of the extract with 3 mL methanol and then by adding 1 mL of DPPH (0.012 g/100 mL). Free radical scavenging activity was calculated as shown in the formula below.

$$\%DPPH^{\cdot} \text{ scavenging} = \left[ \frac{\text{Absorbance of control} - \text{Absorbance of sample}}{\text{Absorbance of control}} \right]$$

As phenolic compounds account for antiradical activity, total phenolic contents in the peat extract were determined using the Folin-Ciocalteu reagent. The reaction mixture contained 100 μl of the extract 500 μl of the Folin-Ciocalteu reagent, and 1.5 ml of 20% sodium carbonate. The final volume was made up to 10 ml with pure water. After 2 h of reaction at ambient temperature, absorbance at 765 nm was measured and used to calculate the phenolic contents using a standard curve prepared with gallic acid.

Peat extract showed average antiradical (DPPH$^{*}$ scavenging) activity of 50,8% and measured average phenolic contents were 0,058 Gallic acid equivalents per ml of extract.

Effect on proliferation of dermal cells

Effect of peat extract, peat humic acids and combination of them on proliferation of dermal fibroblasts was tested using real-time cell monitoring system. The cell proliferation in the presence of test substances was monitored using xCELLigence™ System (Roche, USA). Cells were seeded on xCelligence system compatible E-plate at a density 1000 cells per well and allowed to attach for 24 h before supplementing with test substances. Cells were cultivated for 48 h after adding the extract and/or humic acids. Arbitrary cell index values were measured every 30 minutes of cultivation and normalized to the time point of the addition of glycoprotein fraction. The population doubling times were calculated by RCTA v1.2 software after 24h and 48 h of cultivation. Effect on cellular proliferation was expressed as relative changes of population doubling time compared to control. Fig.1 illustrates the effect of different humic acid concentrations in combination with 0,75% peat ethanol extract on dermal fibroblast proliferation It is seen that combination of 10 μg/ml humic acids with 0,75% peat extract reduces fibroblast population doubling time by up to average 50% within the first 24h and by up to 30% during 48h cultivation.

Free radical scavenging activity together with potential to promote skin cell proliferation makes combination of humic acids and peat extract a valuable anti-ageing cosmetic ingredient. The use of present invention in cosmetic product formulations is better explained by examples given below.

_Example 1 - regenerating facial cream_

| a) | Aqua | 54,95% |
|---|---|---|
| b) | Vitis vinifera (Grape) Seed Oil | 20% |
| | Caprylic/capric triglycerides | 10% |
| | Titanium dioxide | 6% |
| | Olivem-100 (Cetearyl Olivate, Sorbitan Olivate) | 4% |
| | Cetyl alcohol | 2% |
| c) | Humic acids (1mg/ml solution) | 1,00% (final conc. - 10 $\mu$g/ml) |
| | Peat ethanol extract | 0,75% |
| | Tocopherol acetate | 0,5% |
| | Potassium sorbate | 0,4% |
| | Gluconolactone, Sodium Benzoate | 0,4% |
| Ingredient a) is heated to approximately 65°C b) is likewise heated to approximately 65 °C till emulsifying waxes are completely melted, and then added while vigorously stirring to mixture a) to form emulsion. Stirring is continued until the cream has cooled down to approximately 35 °C. Then mixture c) is added while stirring and the cream is homogenized. All concentrations in the table above designated as % are per cents of volume. | | |

_Example 2 - regenerating serum for periocular skin_

| a) | Aqua | 72,42% |
|---|---|---|
| b) | Squalane | 10% |
| | Coco-caprylate | 10% |
| | Olivem-100 (Cetearyl Olivate, Sorbitan Olivate) | 3% |
| | Cetyl alcohol | 2% |
| c) | Humic acids (1 mg/ml solution) | 0,5% (final conc. 5 $\mu$g/ml |
| | Peat ethanol extract | 0,75% |
| | Tocopherol acetate | 0,5% |
| | Potassium sorbate | 0,4% |
| | Gluconolactone, Sodium Benzoate | 0,4% |
| | Ubiquinone | 0,03% |
| Ingredient a) is heated to approximately 65°C and mixture b) is likewise heated to approximately 65 °C till emulsifying waxes are completely melted, and then added while vigorously stirring to mixture a) to form emulsion. Stirring is continued until the formulation has cooled down to approximately 35 °C. Then mixture c) is added while stirring and formulation is homogenized. All concentrations in the table above designated as % are per cents of volume. | | |

_Example 3 - facial cleansing lotion_

| a) | Aqua | 76,95% |
|---|---|---|
| | Xanthan gum | 4% |

(continued)

| | | | |
|---|---|---|---|
| b) | Caprylic/capric triglycerides | 9% |
| | Olivem-800 (Ceteareth-6 Olivate) | 3% |
| | Cetyl alcohol | 2% |
| | Oliwax LC(Cetyl Palmitate, Sorbitan Palmitate, Sorbitan Olivate) | 2% |
| c) | Humic acids (1mg/ml solution) | 1% (final.conc 10 μg/ml) |
| | Peat ethanol extract | 0,75 % |
| | Tocopherol acetate | 0,5% |
| | Potassium sorbate | 0,4% |
| | Gluconolactone, Sodium Benzoate | 0,4% |

Mixture a) is heated to approximately 65°C and mixture b) is likewise heated to approximately 65 °C till emulsifying waxes are completely melted, and then added while vigorously stirring to mixture a) to form emulsion. Stirring is continued until the formulation has cooled down to approximately 35 °C. Then mixture c) is added while stirring and the formulation is homogenized. All concentrations in the table above designated as % are per cents of volume.

References

1. Patent application No. DE 10025622 A1. Production of peat-extracts and peat 'brines' for use in cosmetics such as skin cleansers, face masks or night creams by drying and milling natural peat and treating with solvents or natural brine. 2001. Author: Tannert Utz; Owner: Staatsbad Meinberg Gmbh, Tannert Utz.

2. EP 0281679 B1. Low molecular alkali huminates, process for their preparation and their use. 1988. Inventors: Bernhard Seubert, Helmut Dr. Beilharz, Werner Dr. Fickert, Günter Dr. Jeromin, Ulrich Dr. Spitaler; Applicant: Rütgerswerke Aktiengesellschaft.

3. EP 0533865 B1. Peat-derived bioactive products and pharmaceutical and cosmetic compositions containing them. 1992. Inventors: Tadeusz Gersz, Ryszard Kukla, Stanislawa Ritter, Malgorzata Skrzyszewska, Stanislaw Tolpa, Stanislaw Tomkow: Applicant: Torf Ets.

4. EP 1115408 B1. Humic acid and its use in the treatment of various conditions. 2000. Authors: Johannes Dekker, Constance Elizabeth Medlen; Owner: Enerkom Pty Ltd, Johannes Dekker,Constance Elizabeth Medlen.

5. WO 2004050208 A1. Method of extract composition control in peat extraction, peat extract, and use of peat extract. 2004. Authors: Aromtech Ltd, Vesa-Pekka Judin, Heikki Kallio, Veli-Markku Korteniemi, Petri Maeaettae, Saska Tuomasjukka, Baoru Yang; Owner: Aromtech Ltd, Vesa-Pekka Judin, Heikki Kallio, Veli-Markku Korteniemi, Petri Maeaettae, Saska Tuomasjukka, Baoru Yang.

6. Beer A. M., Junginger H. E., Lukanov J., Sagrochev P. 2003. Evaluation of permeation of peat substances through human skin in vitro. International Journal of Pharmaceutics 253:169-175.

7. Beer A-M., Sagorchev P., Lukanov J. 2002. Isolation of biologically active fractions from the water soluble components of fulvic and ulmic acids from peat. Phytomedicine 9 (7): 659-666.

8. Burges N. A., Hurst H. M., Walkden B. 1964. The phenolic constituents of humic acid and their relation to the ligning of the plant cover. Geochimica et Cosmochimica Acta 28(10-11): 1547-1152, IN1, 1553-1554.

9. Yamada P., Isoda H., Han J. K., Talorete T. P. N., Yamaguchi T., Abe Y. 2007. Inhibitory effect of fulvic acid extract from Canadian sphagnum peat on chemical mediator release by RBL-2H3 and KU812 Cells. Bioscience, Biotechnology and Biochemistry 71 (5): 1294-1305.

10. Yang H-L., Hseu Y-C., Hseu Y-T., Lu F-J., Lin E., Lai J-S. 2004. Humic acid induces apoptosis in human premyelocytic leukemia HL-60 cells. Life Sciences 75: 1817-1831.

11. Orru M., Übner M., Orru H. 2011. Chemical properties of peat in three peatlands with balneological potential in Estonia. Estonian Journal of Earth Sciences, 2011, 60, 1, 43-49 doi: 10.3176/earth.2011.1.04.

12. Pena-Mendez E. M., Havel J., Patočka J. 2005. Humic substances - compounds of still unknown structure: applications in agriculture industry, environment and biomedicine. Journal of Applied Biomedicine 3:13-24.

13. Schepetikin I., Khlebnikov A., Kwon S. B. 2002. Medical Drugs From Humus Matter: Focus on Mumie. Drug Development Research, 57:140-159.

14. Stepanova E. a., Orlov D. S. 1996. Chemical Characterization of Humic Acids from Sapropels.Eurasian SoilScience C/C of Pochvovedenie29 (10): 1107-1112.

**Claims**

1. An anti-ageing cosmetic active ingredient, being a peat derived bioactive product, **characterized in that** it contains peat extract in combination with humic acids isolated from peat.

2. The product according to claim 1, wherein the peat extract is obtained by extraction with 80% ethanol with peat to ethanol ratio 1:100.

3. The product according to claim 1, wherein humic acids obtainable by reacting dry peat with 0.1 M HCl, followed by resuspension of precipitate in water and adjustment of pH to 7 with the addition of 1 M NaOH, after 24 h the alkaline slurry is filtered and the particle free filtrate is acidified to a pH 1, then sediment which contained humic acids is washed with distilled water and centrifuged discarding the supernatant and humic acid dispersion in distilled water is dialyzed against water, resulting humic acids is lyophilized.

4. The product according to any one of the preceding claims, which is **characterized by** capability to promote proliferation of dermal fibroblasts by 35-50% during the first 24 h of incubation.

5. Use of the product according to any one of the preceding claims in anti-ageing cosmetic formulations at concentrations 0,5-1 volume % of peat extract combined with 5-20 µg/ml humic acids.

Fig. 1

Europäisches
Patentamt

European
Patent Office

Office européen
des brevets

# EUROPEAN SEARCH REPORT

Application Number

EP 13 19 4683

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DE 28 50 685 A1 (KNEER FRANZ X) 4 June 1980 (1980-06-04) * page 3 * ----- | 1-4 | INV. A61Q19/08 A61K8/97 A61K35/10 A61K8/96 |
| Y,D | WO 2004/050208 A1 (AROMTECH LTD [FI]; KALLIO HEIKKI [FI]; KORTENIEMI VELI-MARKKU [FI]; TU) 17 June 2004 (2004-06-17) * page 15, line 27 - page 16, line 8 * ----- | 5 | |
| Y | DE 298 22 918 U1 (HORST HANS JOERG DR [DE]) 25 February 1999 (1999-02-25) * claims 1-8 * * page 15 - page 16 * ----- | 5 | |
| A,D | DE 100 25 622 A1 (STAATSBAD MEINBERG GMBH [DE]; TANNERT UTZ [DE]) 29 November 2001 (2001-11-29) * column 2 - column 3; claim 1 * ----- | 1-5 | |
| A | WO 92/16216 A1 (TORF ETS [LI]) 1 October 1992 (1992-10-01) * page 14 - page 15; examples 11,12 * ----- | 1-5 | TECHNICAL FIELDS SEARCHED (IPC) A61Q A61K |

The present search report has been drawn up for all claims

| Place of search | Date of completion of the search | Examiner |
|---|---|---|
| Munich | 24 March 2014 | Bader, Karl Günther |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

    ........................................................................

& : member of the same patent family, corresponding
    document

EPO FORM 1503 03.82 (P04C01)

**ANNEX TO THE EUROPEAN SEARCH REPORT
ON EUROPEAN PATENT APPLICATION NO.**

EP 13 19 4683

This annex lists the patent family members relating to the patent documents cited in the above-mentioned European search report.
The members are as contained in the European Patent Office EDP file on
The European Patent Office is in no way liable for these particulars which are merely given for the purpose of information.

24-03-2014

| Patent document cited in search report | | Publication date | Patent family member(s) | | Publication date |
|---|---|---|---|---|---|
| DE 2850685 | A1 | 04-06-1980 | NONE | | |
| WO 2004050208 | A1 | 17-06-2004 | AU | 2003302574 A1 | 23-06-2004 |
| | | | EP | 1426028 A1 | 09-06-2004 |
| | | | EP | 1567242 A1 | 31-08-2005 |
| | | | WO | 2004050208 A1 | 17-06-2004 |
| DE 29822918 | U1 | 25-02-1999 | NONE | | |
| DE 10025622 | A1 | 29-11-2001 | NONE | | |
| WO 9216216 | A1 | 01-10-1992 | AT | 162077 T | 15-01-1998 |
| | | | DE | 533865 T1 | 04-11-1993 |
| | | | DE | 69224024 D1 | 19-02-1998 |
| | | | DE | 69224024 T2 | 27-08-1998 |
| | | | DK | 0533865 T3 | 14-09-1998 |
| | | | EP | 0533865 A1 | 31-03-1993 |
| | | | ES | 2041616 T1 | 01-12-1993 |
| | | | GR | 3026215 T3 | 29-05-1998 |
| | | | GR | 93300103 T1 | 29-10-1993 |
| | | | HK | 1002930 A1 | 25-09-1998 |
| | | | HU | 217370 B | 28-01-2000 |
| | | | JP | 3759162 B2 | 22-03-2006 |
| | | | JP | H06503835 A | 28-04-1994 |
| | | | LV | 12284 A | 20-06-1999 |
| | | | PL | 298132 A1 | 24-01-1994 |
| | | | US | 5747050 A | 05-05-1998 |
| | | | WO | 9216216 A1 | 01-10-1992 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1115408 B1
- EP 0533865 B1
- EP 0281679 B1
- WO 2004050208 A1
- DE 10025622 A1
- IN 115531554

### Non-patent literature cited in the description

- **BEER A. M. ; JUNGINGER H. E. ; LUKANOV J. ; SAGROCHEV P.** Evaluation of permeation of peat substances through human skin in vitro. *International Journal of Pharmaceutics,* 2003, vol. 253, 169-175
- **BEER A-M. ; SAGORCHEV P. ; LUKANOV J.** Isolation of biologically active fractions from the water soluble components of fulvic and ulmic acids from peat. *Phytomedicine,* 2002, vol. 9 (7), 659-666
- **BURGES N. A. ; HURST H. M. ; WALKDEN B.** The phenolic constituents of humic acid and their relation to the ligning of the plant cover. *Geochimica et Cosmochimica Acta,* 1964, vol. 28 (10-11), 1547-1152
- **YAMADA P. ; ISODA H. ; HAN J. K. ; TALORETE T. P. N. ; YAMAGUCHI T. ; ABE Y.** Inhibitory effect of fulvic acid extract from Canadian sphagnum peat on chemical mediator release by RBL-2H3 and KU812 Cells. *Bioscience, Biotechnology and Biochemistry,* vol. 71 (5), 1294-1305
- **YANG H-L. ; HSEU Y-C. ; HSEU Y-T. ; LU F-J. ; LIN E. ; LAI J-S.** Humic acid induces apoptosis in human premyelocytic leukemia HL-60 cells. *Life Sciences,* 2004, vol. 75, 1817-1831
- **ORRU M. ; ÜBNER M. ; ORRU H.** Chemical properties of peat in three peatlands with balneological potential in Estonia. *Estonian Journal of Earth Sciences,* 2011, vol. 60 (1), 43-49
- **SCHEPETIKIN I. ; KHLEBNIKOV A. ; KWON S. B.** Medical Drugs From Humus Matter: Focus on Mumie. *Drug Development Research,* 2002, vol. 57, 140-159
- **STEPANOVA E. ; ORLOV D. S.** Chemical Characterization of Humic Acids from Sapropels. *Eurasian SoilScience C/C of Pochvovedenie,* 1996, vol. 29 (10), 1107-1112